# EUROPEAN PATENT SPECIFICATION

(11) **EP 4 331 545 B1**
(45) Date of publication and mention of the grant of the patent: **30.07.2025**
(21) Application number: 22815700.4
(22) Date of filing: 30.03.2022
(51) Int. Cl.: A61F 13/15, A61F 13/472, A61F 13/511, A61F 13/53, A61F 13/534, A61L 33/16, A61L 15/38, A61L 15/42

(54) **ABSORBENT ARTICLE FOR ABSORBING MENSTRUAL BLOOD**
ABSORBIERENDER ARTIKEL ZUM ABSORBIEREN VON MENSTRUATIONSBLUT
ARTICLE ABSORBANT POUR ABSORBER LE SANG MENSTRUEL

(30) Priority: 31.05.2021 JP 2021091924
(43) Date of publication of application: 06.03.2024
(73) Proprietor: Unicharm Corporation, Shikokuchuo-shi, Ehime 799-0111 (JP)
(72) Inventor: NANAUMI, Hisataka, Kanonji-shi, Kagawa 769-1602 (JP); IWAMOTO, Makiko, Kanonji-shi, Kagawa 769-1602 (JP)
(74) Representative: Dolleymores
(86) International application number: PCT/JP2022/016112
(87) International publication number: WO 2022/254943

(56) References cited:
- WO-A1-2020/179879
- JP-A- 2007 535 361
- JP-A- 2016 013 976
- JP-A- 2020 147 512

## Description

### FIELD

The present disclosure relates to an absorbent article for absorbing menstrual blood.

### BACKGROUND

Absorbent articles generally used for absorbing menstrual blood, particularly sanitary napkins, are configured to absorb menstrual blood mainly based on a hydrophilic gradient, a sparseness and density gradient, and a capillary force. However, for example when the viscosity of menstrual blood is high, the intended absorbency cannot be exhibited in some cases. Developments have been made to further enhance the function of absorbent articles for absorbing menstrual blood.

Patent Literature 1 discloses an absorbent article having a top sheet containing a blood modifying agent having specific physical properties. According to the literature, the blood modifying agent has an action of decreasing the viscosity and surface tension of blood.

Patent Literature 2 discloses an absorbent article comprising a liquid-permeable sheet (hemagglutinating sheet) containing a hemagglutination agent. According to the literature, the hemagglutinating sheet has an action of agglutinating red blood cells in blood when the hemagglutinating sheet comes into contact with the blood. Patent Literature 3 discloses a substrate treated with a linked enzyme.

### [CITATION LIST]

### [PATENT LITERATURE]

Patent Literature 1: Japanese Unexamined Patent Publication No. 2013-63245
Patent Literature 2: Japanese Unexamined Patent Publication No. 2017-217066
Patent Literature 3: United States Patent Application Publication No. US20050256471

### SUMMARY

### [TECHNICAL PROBLEM]

Conventional absorbent articles cannot exhibit sufficient absorbency depending on the state of menstrual blood in some cases. In particular, menstrual blood may contain highly viscous menstrual blood of high viscosity (so-called "sludgy menstrual blood"). Since the highly viscous menstrual blood is, for example, solid, liver-like, jelly-like or gel-like, the menstrual blood is less likely to be diffused and absorbed into the sheet of the absorbent article having a dense configuration. When highly viscous menstrual blood remains on the surface of the absorbent article without being diffused and absorbed, there arise problems such as leakage, anxiety of leakage, stickiness to the skin, and repulsive appearance. Further, when the diffusion and absorption of the menstrual blood are inhibited by the highly viscous menstrual blood in the absorbent article, there arise problems such as leakage and stickiness.

An object of the present disclosure is to provide an absorbent article having improved menstrual blood absorbency.

### [SOLUTION TO PROBLEM]

An invention as defined in claim 1.

### [ADVANTAGEOUS EFFECT OF THE INVENTION]

According to the present invention, an absorbent article having improved menstrual blood absorbency can be provided. In particular, according to the present invention, since the "highly viscous menstrual blood" in the absorbent article can be reduced, the diffusion and absorption of menstrual blood in the absorbent article can be promoted.

### BRIEF DESCRIPTION OF DRAWINGS

FIG. 1 is a plan schematic view of a sanitary napkin 1 as an absorbent article according to the present disclosure.
FIG. 2 is a photograph showing a napkin according to Example 2 and simulated highly viscous menstrual blood arranged on the napkin.
FIG. 3 is a photograph of the napkin according to Example 2 after the simulated highly viscous menstrual blood was left to stand on the napkin.
FIG. 4 is a photograph showing a napkin according to Example 4 and actual highly viscous menstrual blood arranged on the napkin.
FIG. 5 is a photograph of the napkin according to Example 4 after the highly viscous menstrual blood was left to stand on the napkin.

### DESCRIPTION OF EMBODIMENTS

The present inventors have achieved the present invention by paying attention to the fact that "highly viscous menstrual blood" is caused by the progression of blood coagulation in menstrual blood.

During menstruation, the functional layer of the endometrium is developed. During the premenstrual period, the necrosis of the functional layer of the endometrium proceeds, and then during menstruation, the functional layer falls off from the uterus and becomes menstrual blood. The menstrual blood comprises endometrium and blood.

Without intending to be limited by theory, during the premenstrual period, the properties of blood in the endometrium is changed so as to suppres blood coagulation, and particularly, the amount of plasmin is increased. Plasmin has a role of maintaining the liquid state of menstrual blood by degrading fibrin which promotes blood coagulation. Due to the necrosis of the functional layer of the endometrium during the premenstrual period, coagulation progresses by blood coagulation factors in the blood comprised in the menstrual blood. However, normally, blood coagulation is suppressed and/or the coagulated blood is liquefied by the action of the plasmin whose amount is increased during the premenstrual period. Further, due to such blood coagulation and the action of plasmin that inhibits the blood coagulation, in the menstrual blood, a significant reduction or deficiency of blood coagulation factors (in particular, activated factor XIII) occurs, and blood coagulation is much less likely to occur. However, it is considered that, in a case where the amount of plasmin is relatively low due to physical constitution and physical condition, etc., or in a case where the amount of menstrual blood is relatively large, the highly viscous menstrual blood occurs due to insufficient degradation of fibrin by plasmin.

Since the absorbent article according to the present disclosure has a functional substance having a function of inhibiting a factor that acts on blood coagulation, coagulated blood can be reduced in the menstrual blood, and due to that, highly viscous menstrual blood can be effectively reduced in the menstrual blood.

The present disclosure includes the following.

An absorbent article for absorbing menstrual blood, having a functional substance in accordance with claim 1.

The absorbent article has a functional substance, and the functional substance has a function of inhibiting a factor that acts on blood coagulation. The "highly viscous menstrual blood" occurs through the progression of blood coagulation in the menstrual blood. Therefore, according to the absorbent article, when the menstrual blood is excreted in the absorbent article, the "highly viscous menstrual blood" in the absorbent article can be reduced, and/or the occurrence of the highly viscous menstrual blood in the absorbent article can be suppressed .

The functional substance of the absorbent article has a function of suppressing blood coagulation in the menstrual blood and/or a function of promoting lowering of viscosity of coagulated blood contained in the menstrual blood.

The absorbent article has the functional substance that inhibits a factor that acts on blood coagulation, and this functional substance can suppress blood coagulation in the menstrual blood and/or promote the lowering of the viscosity of coagulated blood contained in the menstrual blood. Therefore, according to the absorbent article, when the menstrual blood is excreted in the absorbent article, the "highly viscous menstrual blood" in the absorbent article can be reduced, and/or the occurrence of the highly viscous menstrual blood in the absorbent article can be suppressed.

It should be noted that the lowering of the viscosity of the coagulated blood particularly means that the solubility of the coagulated blood is increased or the coagulated blood is liquefied. The coagulated blood of which the viscosity is lowered exhibits enhanced diffusibility and/or mobility in an absorbent article.

The functional substance contains an enzyme having fibrin degradation ability.

Without intending to be limited by theory, fibrin (particularly fibrin polymers and stabilized fibrin) present in the blood forms a mesh-like membrane, and this membrane aggregates red blood cells, platelets, and the like to coagulate the blood. The absorbent article has the enzyme having fibrin degradation ability (fibrinolytic ability). Therefore, when the menstrual blood is excreted in the absorbent article, the lowering of the viscosity of the coagulated blood contained in the menstrual blood can be promoted and blood coagulation can be suppressed by degrading the fibrin in the menstrual blood. Due to that, problems caused by the highly viscous menstrual blood can be solved or reduced.

The absorbent article may have an auxiliary component capable of enhancing the fibrin degradation ability of the enzyme.

The absorbent article having the auxiliary component is capable of enhancing the function of the enzyme having fibrin degradation ability. Therefore, when the menstrual blood is excreted in the absorbent article, the lowering of the viscosity of the coagulated blood contained in the menstrual blood can be promoted particularly effectively, and blood coagulation can be suppressed particularly effectively. Due to that, problems caused by the highly viscous menstrual blood can be solved or reduced.

The enzyme having the fibrin degradation ability may be nattokinase.

Nattokinase is a serine protease that exhibits a higher fibrin degradation ability than plasmin. Therefore, when the menstrual blood is excreted in the absorbent article, the lowering of the viscosity of the coagulated blood contained in the menstrual blood can be promoted particularly effectively, and blood coagulation can be suppressed particularly effectively. Due to that, problems caused by the highly viscous menstrual blood can be solved or reduced.

The functional substance may contain a compound having an antiplatelet action.

When platelets present in the blood are activated, the platelets aggregate together to coagulate the blood. An absorbent article having a compound having an antiplatelet action may be provided. Therefore, when the menstrual blood is excreted in the absorbent article, blood coagulation in the menstrual blood can be suppressed, and due to that, problems caused by the highly viscous menstrual blood can be solved or reduced.

The functional substance may be solid or pasty.

Since the functional substance may be present in the form of solid or paste in the absorbent article, the functional substance is less likely to move in the absorbent article, for example as compared with a case where the functional substance is in the form of liquid. In addition, in a case where the solid or pasty functional substance contains an enzyme or the like, after contact with the menstrual blood, the enzyme or the like is dissolved in the liquid component contained in the menstrual blood, and thus the enzyme can exert its function well.

The absorbent article may be configured so that, when the absorbent article is put on, the functional substance contained in the absorbent article is arranged in an excretion opening contact region.

A portion that faces the excretion opening of the wearer is the first portion of the absorbent article that comes into contact with the most menstrual blood. An absorbent article may have the functional substance in the excretion opening contact region, the highly viscous menstrual blood contained in the excreted menstrual blood can be rapidly reduced, and thereby the absorption of the menstrual blood into the absorbent article is promoted, and/or the occurrence of the highly viscous menstrual blood in the absorbent article can be suppressed at an early stage.

An absorbent article may comprise a liquid-permeable top sheet and an absorbent core, wherein
at least any one of the top sheet and the absorbent core has the functional substance.

Since an absorbent article has the functional substance in at least any one of the top sheet and the absorbent core, the absorbent article has enhanced permeability and/or absorbency for the menstrual blood having highly viscous menstrual blood.

An absorbent article, wherein
the top sheet has the functional substance.

Since the top sheet of an absorbent article has the functional substance, the highly viscous menstrual blood in the top sheet is reduced, and due to that, the diffusion of the menstrual blood on and in the top sheet and/or the movement of the menstrual blood in a direction from the top sheet toward the absorbent core is promoted.

The absorbent article, wherein
the absorbent core has the functional substance.

Since the absorbent core has the functional substance, the highly viscous menstrual blood in the absorbent core is reduced, and due to that, the diffusion (and absorption) of the menstrual blood on and in the absorbent core is promoted.

An absorbent article, further comprising a second sheet arranged between the top sheet and the absorbent core, wherein
the second sheet has the functional substance.

Since the second sheet has the functional substance, the highly viscous menstrual blood in the second sheet is reduced, and due to that, the diffusion of the menstrual blood on and in the second sheet and/or the movement of the menstrual blood in a direction from the second sheet toward the absorbent core is promoted.

Hereinafter, the present invention and its constituent elements will be described in more detail.

### <Factor That Acts on Blood Coagulation>

A factor that acts on blood coagulation has, in particular, a function of coagulating blood and/or a function of maintaining a coagulation state of the coagulated blood in menstrual blood. Examples of the factor that acts on blood coagulation include fibrin (particularly fibrin monomer, fibrin polymer and/or stabilized fibrin), blood coagulation factors, platelets, and platelet aggregation promoting substances. The blood coagulation factor in particular includes thrombin, activated factor II, activated factor VII, activated factor IX, activated factor X, and activated factor XIII. Further, the platelet aggregation promoting substance in particular include TXA₂ and cAMP.

### <Functional Substance>

The functional substance according to the present disclosure has a function of inhibiting a factor that acts on blood coagulation. The functional substance includes an enzyme having fibrin degradation ability, a substance having an antiplatelet action (particularly, compounds), a factor that promotes the activation of plasmin, and a coagulation inhibitor.

Among these, the enzyme having fibrin degradation ability and the factor that promotes the activation of plasmin mainly have an action of promoting the lowering of the viscosity of coagulated blood. In addition, the substance having an antiplatelet action and the coagulation inhibitor mainly have a function of suppressing blood coagulation.

### <Enzyme Having Fibrin Degradation Ability>

The enzyme having fibrin degradation ability may be a protease, particularly a serine protease or a cysteine protease (not within the scope of the claims). In particular, the enzyme having fibrin degradation ability can cleave fibrin polymers and/or stabilized fibrin.

Without intending to be limited by theory, in the menstrual blood, through the action of the blood coagulation factors comprising a series of molecules, the production of fibrin monomers from fibrinogen, polymerization of fibrin monomers, and crosslinking (stabilization) of fibrin progress, to coagulate blood. Fibrin polymers and stabilized fibrin form a mesh-like structure, and with this structure, red blood cells, platelets, and the like are aggregated to coagulate blood. The enzyme having fibrin degradation ability can particularly suppress blood coagulation by degrading the fibrin polymers and the stabilized fibrin, and can promote the lowering of the viscosity of coagulated blood by degrading the fibrin polymers and the stabilized fibrin forming the mesh-like structure.

The enzyme having fibrin degradation ability includes plasmin, nattokinase, DFE27, Subtilisin DFE, Subtilisin QK-2, bromelain (not within the scope of the claims), and serrapeptase.

### (Plasmin)

Plasmin is an enzyme (serine protease) that has the ability to degrade fibrin (fibrinolytic ability) and is usually present in menstrual blood. Plasmin is capable of degrading fibrin. The degradation of fibrin by plasmin produces a degradation product called D-dimer and other degradation products.

### (Nattokinase)

Nattokinase is an enzyme (serine protease) having an ability to degrade fibrin. Nattokinase is generally contained in natto and can be produced by Bacillus subtilis natto. Nattokinase can mainly degrade fibrin to DD (175 kDa) and LD (110 kDa). Without intending to be limited by theory, it is considered that nattokinase has a three-dimensional structure adapted for degrading fibrin, and degrades fibrin by a mechanism similar to that of plasmin. Nattokinase has been reported to have an enzyme specificity constant for fibrin that is approximately six times that of plasmin. This indicates that nattokinase has a higher fibrinolytic action than plasmin.

Without intending to be limited by theory, nattokinase exhibits particularly high activity under the conditions of a temperature of 30°C to 40°C and a pH of 6 to 9. When an absorbent article, in particular a sanitary napkin, is put on, the temperature thereof become around 35°C due to body temperature, and the pH of menstrual blood is around 7; therefore, it is considered that nattokinase functions particularly well under the conditions of use of the absorbent article for absorbing menstrual blood.

Further, although not intending to be limited by theory, according to the simulation result on the binding mode of fibrinogen and nattokinase, it is considered that nattokinase interacts with fibrinogen through eight amino acid residues (Gly61, Ser63, Thr99, Phe189, Leu209, Tyr217, Asn218, and Met222) of nattokinase, and performs degradation of fibrin mainly through Ser221. It is considered that by utilizing such detailed knowledge about the mechanism of the action of nattokinase, the blood coagulation inhibitory action and the coagulated blood liquefying action can be further enhanced in the absorbent article of the present invention. Specifically, for example, nattokinase may be fixed on a base material so that the active site of nattokinase easily comes into contact with highly viscous menstrual blood.

Nattokinase is highly safe for the human body. Therefore, in a case where nattokinase is used as the functional substance in an absorbent article, an absorbent article having particularly excellent safety can be obtained.

Nattokinase is commercially available, for example, in the form of powder. For example, Nattokinase having a particle size of 15 to 35 µm (manufactured by FUJIFILM Wako Pure Chemical Corporation) is commercially available.

### (DFE27)

DFE27 is an enzyme (serine protease) having an ability to degrade fibrin. DFE27 is generally contained in Douchi and can be produced by B. subtilis DC27 (Bacillus subtilis DC27).

### (Subtilisin DFE)

Subtilisin DFE is an enzyme (serine protease) having an ability to degrade fibrin. Subtilisin DFE is generally contained in Douchi and can be produced by B. amyloliquefaciens DC-4.

### (Subtilisin QK-2)

Subtilisin QK-2 is an enzyme (serine protease) having an ability to degrade fibrin. Subtilisin QK-2 is generally contained in fermented soybean, and can be produced by B. subtilis QK02.

### (Bromelain)

Bromelain is an enzyme (cysteine protease) having an ability to degrade fibrin that does not fall within the scope of the claims. The bromelain is included in pineapple fruit and the like.

### (Serrapeptase)

Serrapeptase is an enzyme having an ability to degrade fibrin. Serrapeptase is generally contained in silkworms and can be produced by non-pathogenic bacteria Serratia E15.

### (Auxiliary Component)

The auxiliary component, which is capable of enhancing the fibrin degradation ability (fibrinolytic ability) of the enzyme having fibrin degradation ability, includes fatty acids having 12 to 18 carbon atoms, ground vegetables, EPA, DHA, spices, zinc ions (Zn²⁺), manganese ions (Mn²⁺), calcium ions (Ca²⁺), and potassium ions (K⁺).

The fatty acid having 12 to 18 carbon atoms can particularly enhance the fibrinolytic ability of nattokinase. It is preferable that the fatty acid having 12 to 18 carbon atoms is used at a blending ratio of 4 to 12 mg/2000 FU with respect to nattokinase.

The ground vegetable, EPA, and DHA can particularly enhance the fibrinolytic ability of nattokinase. The ground vegetable includes, for example, onion powder.

The spice can particularly enhance the fibrinolytic ability of nattokinase. The spice includes chili pepper, in particular powdered chili pepper.

The zinc ions (Zn²⁺) can particularly enhance the fibrinolytic ability of nattokinase. In addition, the manganese ions (Mn²⁺), calcium ions (Ca²⁺), and potassium ions (K⁺) can particularly enhance the fibrinolytic ability of protease derived from Douchi.

### (Coating)

The enzyme (for example, nattokinase) applied to the absorbent article can be subjected to a coating treatment. In this case, the fibrinolytic ability of the enzyme having fibrin degradation ability may be maintained particularly well. Further, the behavior, properties, shape, and the like of the enzyme can be adjusted by such a coating treatment, and due to that, in addition to maintaining the above-mentioned fibrinolytic ability, effects such as sustained release, moisture absorption inhibition, deterioration prevention, stickiness prevention, and the like can be obtained. The coatings include oil and fat coatings and ceramic coatings.

### (Fixation (Immobilization))

The enzyme can be fixed to the absorbent article (in particular to a material constituting the absorbent article) by a substance that promotes the immobilization of the enzyme having fibrin degradation ability in the absorbent article, in order to further enhance the activity of the enzyme. The substance that promotes such immobilization includes an amphiphilic molecule.

### <Factor That Promotes Activation of Plasmin>

As the functional substance, a factor that promotes the activation of plasmin can also be used. Normally, plasmin is present in menstrual blood and can degrade fibrin. Accordingly, by using the factor that promotes the activation of plasmin, it is possible to promote the lowering of the viscosity of the highly viscous menstrual blood. The factor that promotes the activation of plasmin includes a factor having an action of inhibiting the activity of a lysis inhibitor PAI-1. As the factor having an action of inhibiting the activity of the lysis inhibitor PAI-1, mention may be made of the above-mentioned nattokinase. Further, bromelain and DFE27 also have an action of promoting the conversion of plasminogen to plasmin.

When a lysis accelerator t-PA is activated, plasmin is produced from plasminogen. t-PA is inactivated by binding to the lysis inhibitor PAI-1. Without intending to be limited by theory, it is considered that by inhibiting the activity of the lysis inhibitor PAI-1, the inactivation of t-PA is suppressed and thus the production of plasmin from plasminogen by t-PA is promoted. It should be noted that plasmin is inactivated in vivo by binding to a plasmin inhibitor (antiplasmin).

### <Compound Having Antiplatelet Action>

The compound having an antiplatelet action includes bromelain, acetylsalicylic acid (Aspirin (trademark), Bufferin (trademark), and the like), PGI2 derivative beraprost, eicosapentaenoic acid (EPA), docosahexaenoic acid (DHA), and NO-related preparations (for example, nitroglycerin). As other compounds having an antiplatelet action, mention may also be made of clopidogrel sulfate, prasugrel hydrochloride, ticlopidine hydrochloride, ticagrelor, cilostazol, and sarpogrelate hydrochloride.

Generally, when platelets are activated in blood due to factors such as the breakage of the blood endothelium or contact with collagen, aggregation of the platelets occurs. The aggregation of platelets is a reversible reaction. It is considered that by using the compound having an antiplatelet action, blood coagulation can be suppressed.

Without intending to be limited by theory, it is considered that acetylsalicylic acid has an action of irreversibly deactivating cyclooxygenase (COX) that acts on the production of TXA₂, which is a platelet aggregation promoting substance, and thereby exhibits an antiplatelet action.

Further, without intending to be limited by theory, it is considered that PGI2 derivative beraprost suppresses the aggregation of platelets by increasing the synthesis of PGI₂, which is a substance that suppresses the aggregation of platelets.

Further, without intending to be limited by theory, it is considered that EPA and/or DHA produces TXA₃ having no platelet aggregation action from platelets, and due to that, the aggregation of platelets is suppressed.

Fibrin degradation products produced by the enzyme having fibrin degradation ability (particularly, nattokinase) also have a platelet aggregation inhibitory action. The fibrin degradation products produced by nattokinase includes DD (175 kDa) and LD (110 kDa).

### <Coagulation Inhibitor>

As the functional substance, a coagulation inhibitor having a fibrin production inhibitory action can also be used. As such a coagulation inhibitor, mention may be made of bromelain, EDTA, heparin, sodium citrate, warfarin, and sodium fluoride.

Without intending to be limited by theory, in the blood contained in the menstrual blood, the coagulation progresses by the blood coagulation factors. For example, among the blood coagulation factors, thrombin produces fibrin monomers from fibrinogen. In addition, among the blood coagulation factors, activated factor XIII stabilizes the structure composed of fibrin by crosslinking the molecules of the fibrin polymer, and stabilized fibrin is produced.

The coagulation inhibitor can inhibit blood coagulation by inhibiting the action of the blood coagulation factor.

Without intending to be limited by theory, for example, it is considered that bromelain suppresses the conversion of fibrinogen to fibrin by providing a significant extension of the prothrombin time (PT) and the activated partial thromboplastin time (APTT).

Further, without intending to be limited by theory, for example, it is considered that EDTA inhibits the activation of thrombin required for the production of fibrin, by chelating calcium ions required for the activity of thrombin. It is considered that sodium citrate also acts by a similar mechanism.

Further, without intending to be limited by theory, for example, it is considered that warfarin suppresses the activation of thrombin required for the production of fibrin, by inhibiting vitamin K which is required for the action of factors II, VII, IX, and X, which are the blood coagulation factors and act on the activation of thrombin.

Further, without intending to be limited by theory, for example, it is considered that heparin suppresses the activation of thrombin required for the production of fibrin, by enhancing the action of antithrombin that inhibits thrombin.

Nattokinase and serrapeptase also have a fibrin production inhibitory action. Without intending to be limited by theory, it is considered that nattokinase present in blood exhibits a fibrin inhibitory action, for example through the reduction of activated factor XIII that crosslinks fibrin to each other and/or the inhibition of thrombin activity required for the production of fibrin.

### <<Absorbent Article>>

The absorbent article according to the present disclosure has a function of absorbing menstrual blood, and is, for example, a sanitary napkin, a panty liner, or a tampon. The materials that constitute the absorbent article include a top sheet, a back sheet, a second sheet, and an absorbent core.

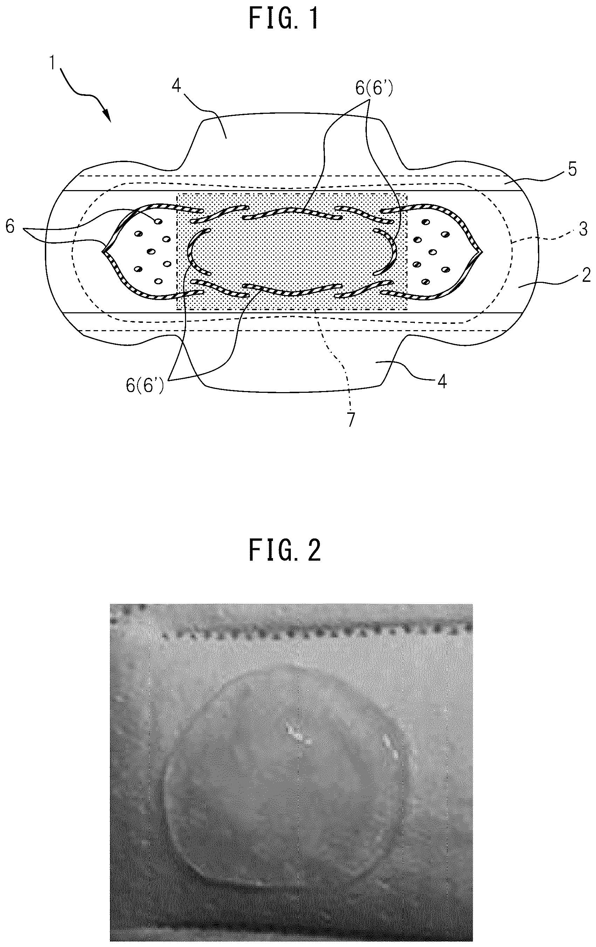

FIG. 1 is a plan schematic view of a sanitary napkin 1 as an absorbent article according to the present disclosure. FIG. 1 is a view of a top sheet 2 observed from a skin side surface thereof. The sanitary napkin 1 has a liquid-permeable top sheet 2, a liquid-impermeable back sheet (not shown), and an absorbent core 3 arranged therebetween. The sanitary napkin 1 shown in FIG. 1 comprises, on two side portions in a lengthwise direction thereof, a pair of side flaps 4 for fixing the sanitary napkin 1 to clothing of a wearer, for example, underpants. The excretion opening contact region is a region defined by four embossments 6' in an application region 7 of the functional substance, and the entire excretion opening contact region of the top sheet 2 includes the application region 7 of the functional substance.

It should be noted that, although the sanitary napkin 1 shown in FIG. 1 has a side sheet 5 and a plurality of embossments 6, an absorbent article according to another embodiment of the present disclosure does not have a side sheet and/or embossments.

In the sanitary napkin 1 shown in FIG. 1, at least an excretion opening contact region of the top sheet 2 has the functional substance according to the present disclosure.

### <Top Sheet>

As the liquid-permeable top sheet, any liquid-permeable top sheet that is commonly used in the art can be employed without any particular limitations, and for example, it is possible to use a sheet-like material having a structure that allows permeation of liquids, such as a porous film, woven fabric, nonwoven fabric or the like.

The fibers constituting the woven fabric and the nonwoven fabric include natural fibers and chemical fibers. The natural fibers, for example, include cellulose such as pulverized pulp and cotton. The chemical fibers include, for example, regenerated cellulose such as rayon and fibril rayon, semi-synthetic cellulose such as acetate and triacetate, thermoplastic hydrophobic chemical fibers, and thermoplastic hydrophobic chemical fibers subjected to a hydrophilization treatment.

The thermoplastic hydrophobic chemical fibers include, for example, single fibers such as polyethylene (PE), polypropylene (PP), and polyethylene terephthalate (PET), and fibers consisting of PE and PP graft polymers.

Examples of the nonwoven fabric include, for example, air-through nonwoven fabrics, spunbond nonwoven fabrics, point bond nonwoven fabrics, spun-lace nonwoven fabrics, needle punched nonwoven fabrics, meltblown nonwoven fabrics, and combinations thereof (for example, SMS).

### <Absorbent Core>

The constituent element of the absorbent core include, for example, hydrophilic fibers, which include, for example, cellulose such as pulverized pulp and cotton, regenerated cellulose such as rayon and fibril rayon, semi-synthetic cellulose such as acetate and triacetate, particulate polymers, fibrous polymers, thermoplastic hydrophobic chemical fibers, hydrophilicized thermoplastic hydrophobic chemical fibers, and combinations thereof. Further, the constituent element of the absorbent core also includes a superabsorbent polymer such as granular matter of a sodium acrylate copolymer or the like.

The absorbent core may be covered with a core wrap. The core wrap is not particularly limited as long as the core wrap is a liquid-permeable material having a barrier property that does not allow permeation of the polymer absorbent body, and examples thereof include a woven fabric or a nonwoven fabric. The woven fabric and the nonwoven fabric include, for example, natural fibers, chemical fibers, tissue, and the like.

The absorbent core may be formed of an absorbent sheet or a polymer sheet, and the thickness thereof is preferably 0.3 to 5.0 mm. As the absorbent sheet or polymer sheet, any sheet can be used without any particular limitation as long as the sheet is usually used in an absorbent article such as a sanitary napkin.

### <Back Sheet>

The absorbent article according to the present disclosure can further comprise a liquid-impermeable back sheet. The back sheet includes a film composed of PE, PP, or the like, a resin film having breathability, a sheet obtained by joining a resin film having breathability to a nonwoven fabric such as spunbond or spunlace, a multilayer nonwoven fabric such as SMS, and the like. In consideration of the flexibility of the absorbent article, a low-density polyethylene (LDPE) film having a basis weight of, for example, about 15 to about 30 g/m² is preferable.

### <Second Sheet>

The absorbent article according to the present disclosure may further comprise a second sheet between the top sheet and the absorbent core. The second sheet can have liquid permeability. As the second sheet, mention may be made of the same examples as described above with respect to the liquid-permeable top sheet.

### <<Absorbent Article Having Functional Substance>>

As described above, the absorbent article according to the present disclosure has a functional substance. In particular, in the absorbent article, the top sheet, the absorbent core, and/or the second sheet constituting the absorbent article may have the functional substance.

### (Application Site)

The functional substance can be present at any location, and for example, the functional substance can be present on the entire surface, or on the central region in the vicinity of the vaginal opening, of the top sheet, the absorbent core, and/or the second sheet in the planar direction. Preferably, when the absorbent article according to the present disclosure is put on, the functional substance comprised in the absorbent article is arranged in the excretion opening contact region.

### (Application Mode)

In a case where the liquid-permeable top sheet is formed of a nonwoven fabric or woven fabric, it is preferable that the functional substance does not block the voids between the fibers of the nonwoven fabric or woven fabric, and for example, the functional substance can be attached as droplets or particulates on the surface of the nonwoven fabric fibers, or cover the surfaces of the fibers. On the other hand, in a case where the liquid-permeable top sheet is formed of a porous film, it is preferable that the functional substance does not block the pores of the porous film, and for example, the functional substance can be attached as droplets or particulates on the surface of the porous film. If the functional substance blocks the voids between the fibers of the nonwoven fabric or woven fabric or the pores of the porous film, the diffusion and/or movement of liquid in the absorbent article may be inhibited.

In particular, the functional substance is present in the form of solid or paste in the absorbent article. As a solvent for obtaining the functional substance in the form of paste, for example, an amphiphilic molecule can be used. It is preferable that the functional substance has a larger surface area, and it is preferable that the functional substance that is present in the form of particulates has a smaller particle diameter.

### (Applicable Amount)

The amount of the functional substance to be applied in the absorbent article can be appropriately set according to the type of the functional substance and the like. For example, in a case where the functional substance is an enzyme having fibrin degradation ability (for example, nattokinase), it is preferable that the functional substance is present on the surface of the top sheet, for example, in an amount in a range of 0.1 to 200 g/m², 0.5 to 100 g/m², 1.0 to 75 g/m², 2.0 to 50 g/m², or 5.0 to 25 g/m². If the amount of the functional substance is too small, the effect of the functional substance on blood coagulation may not be sufficient in some cases, and if the amount of the functional substance is too large, the absorbency of the absorbent article may be decreased in some cases.

In a case where the material (for example the top sheet) to which the functional substance is applied is a nonwoven fabric or a porous film formed of a synthetic resin, it is preferable that the material is subjected to hydrophilization treatment by coating or mixing with a hydrophilic agent. When the original material is hydrophilic, a lipophilic modifying agent having an IOB of about 0.00 to 0.60 and high organicity is applied to the material, and thus there will be created sparsely dispersed lipophilic regions and hydrophilic regions. Therefore, it is considered that this allows consistent absorption performance to be exhibited for menstrual blood which consists of hydrophilic components (blood plasma, and the like) and lipophilic components (blood cells, and the like).

### <Manufacturing of Absorbent Article>

The absorbent article according to the present disclosure can be manufactured by a method comprising a step of applying the functional substance to the absorbent article. The way for applying the functional substance to the absorbent article is not particularly limited. For example, a pasty functional substance or a functional substance prepared in a solution or dispersion can be coated or sprayed to the absorbent article, in order to apply the functional substance. It may also be possible to arrange powdery functional substance directly on the material constituting the absorbent article.

### (Solvent and Dispersion Medium)

When the functional substance is applied to the absorbent article, a solvent or dispersion medium for dissolving or dispersing the functional substance can be appropriately selected according to the properties of the functional substance, and it is preferable that the solvent or dispersion medium does not inhibit the function of the functional substance. Such a solvent or dispersion medium includes water and physiological saline.

After the functional substance is applied to the absorbent article, drying may be appropriately performed, for example, at room temperature, which makes it possible to remove, for example, the solvent or the dispersion medium.

The method for applying the functional substance is not particularly limited, and the functional substance may be heated as necessary. For example, the functional substance can be applied by dipping or gravure coating, and can be applied by a non-contact type coater such as a spiral coater, a curtain coater, a spray coater, a dip coater, or the like, and can be applied by a contact type coater such as a roll coater, a gravure coater, or the like. From the viewpoint that the functional substance is uniformly dispersed throughout, and from the viewpoint that the material of the absorbent article is not damaged, a non-contact type coater is preferable. Further, the functional substance can also be applied using a control seam HMA gun. By increasing the air pressure of the control seam HMA gun, the solution or dispersion of the functional substance can be applied in the form of fine particulates. It should be noted that when the functional substance is applied, in a case where the heating is performed for a relatively long period of time (for example, 30 minutes or longer), it is preferable that the heating temperature is relatively low (for example, 50°C or lower), which makes it possible to retain the function of the functional substance particularly well.

The functional substance can be applied (for example, coated or sprayed) in manufacturing the material (for example, a nonwoven fabric) of the top sheet, or can also be applied in the manufacturing line for manufacturing the absorbent article. From the viewpoint of minimizing equipment investment, it is preferable that the functional substance is applied in the manufacturing line of the absorbent article, and in order to prevent shedding of the functional substance which may contaminate the line, it is preferable that the functional substance is applied during a step downstream from the manufacturing line, and specifically, immediately before the product is enclosed in an individual package.

### Examples

Hereinafter, the present disclosure will be described with reference to examples, but the present disclosure is not limited to these examples.

### <<Example 1>>

In Example 1, the ability of the functional substance to liquefy coagulated blood is evaluated. In Example 1, solidified menstrual blood containing coagulated blood and endometrial fragments was used. To this solidified menstrual blood, a solution of plasmin (Plasmin, manufactured by FUJIFILM Wako Pure Chemical Corporation) as a functional substance was added dropwise, and the solution was left to stand for 20 minutes. The plasmin solution had a concentration of 2 mg/mL, and, as a solvent, "100 mmol/L sodium phosphate buffer (pH 7.3) (containing 1 mmol/L 6-aminohexanoic acid and 25% glycerol)" was used. As a result, the solidified menstrual blood was liquefied. It should be noted that in the liquefied menstrual blood, fragments presumed as endometrial cells were observed. The results are shown in Table 1 below.

### <<Comparative Example 1>>

An experiment was conducted in the same manner as in Example 1, except that physiological saline is used instead of the plasmin solution. Even 20 minutes after the dropwise addition of physiological saline, the solidified menstrual blood was not liquefied and no change was observed. The results are shown in Table 1 below.

### [Table 1]

**Table 1**

| | Object | Functional substance | Result |
|---|---|---|---|
| Example 1 | Solidified menstrual blood | Plasmin | Liquefied |
| Comparative Example 1 | Solidified menstrual blood | - | Not liquefied (no change) |

The results of Example 1 and Comparative Example 1 show that the viscosity of coagulated blood can be lowered by using the substance having an action of degrading fibrin which is a coagulation factor substance.

### <<Examples 2 to 3 and Comparative Example 2>>

In Examples 2 and 3 and Comparative Example 2, the performance of the functional substance was evaluated using simulated highly viscous menstrual blood as the coagulated blood contained in the menstrual blood.

### (Simulated Highly Viscous Menstrual Blood)

Simulated highly viscous menstrual blood can be prepared by mixing fibrinogen and thrombin in a liquid (particularly, in a physiological saline) to produce fibrin. Since it is not easy to obtain highly viscous menstrual blood in actual menstrual blood collected from a human, the use of the simulated highly viscous menstrual blood makes it possible to effectively proceed the verification work for the dissolution and degradation of highly viscous menstrual blood.

### <Example 2>

### (Dropping Test)

In Example 2, a 4-mL simulated highly viscous menstrual blood was prepared in a petri dish by mixing fibrinogen (Fibrinogen, manufactured by FUJIFILM Wako Pure Chemical Corporation) and thrombin (Thrombin, manufactured by FUJIFILM Wako Pure Chemical Corporation) in a physiological saline as a solvent. A solution was prepared by dissolving granular Nattokinase (particle size: 15 to 35 µm, FUJIFILM Wako Pure Chemical Corporation) as a functional substance in a physiological saline at a concentration of 6%. Then, 2 mL of this solution was added dropwise onto the simulated highly viscous menstrual blood in the above-mentioned petri dish, left to stand, and the time required for the simulated highly viscous menstrual blood to be completely liquefied was measured. As a result, the time required for the liquefaction of the simulated highly viscous menstrual blood was 10 minutes. The results are shown in Table 2 below.

### (Napkin Test)

To a region of 3 cm × 3 cm of the central portion of the top sheet of a sanitary napkin (Body Fit Regular (trade name), manufactured by Unicharm Corporation), a physiological saline solution of Nattokinase prepared at a concentration of 27% was uniformly sprayed. The amount of Nattokinase sprayed and attached to the top sheet was 12 gsm (= 12 g/m²). The napkin sprayed with the solution was left to stand at room temperature for 120 minutes to dry, thereby producing a test napkin.

2.5 mL of the simulated highly viscous menstrual blood was arranged in a region of the surface of the obtained test napkin in which Nattokinase was sprayed. The state immediately after the arrangement is shown in the photograph of FIG. 2.

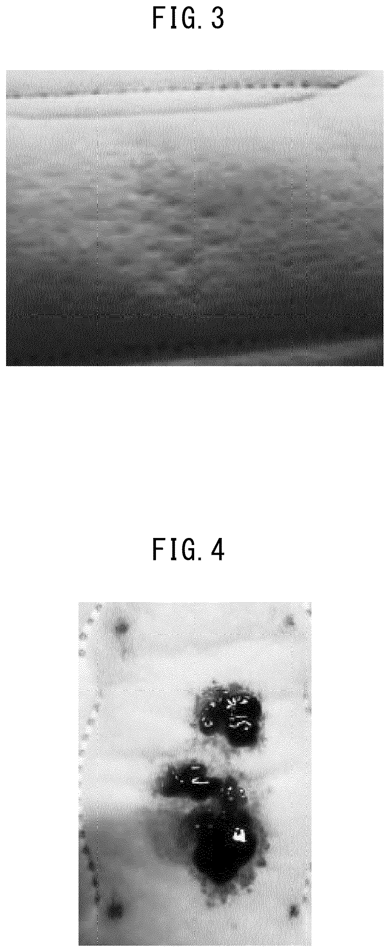

Thereafter, the napkin was left to stand under the conditions of a temperature of 35°C and a humidity of 60% for 40 minutes. The state of the napkin after standing is shown in FIG. 3. The amount of the simulated highly viscous menstrual blood remaining on the napkin was measured, and the remaining amount was 0.03 g. The results are shown in Table 2 below.

### <Example 3>

### (Dropping Test)

A dropping test was conducted in the same manner as in Example 2, except that a physiological saline solution of Plasmin (manufactured by FUJIFILM Wako Pure Chemical Corporation) was used instead of Nattokinase. As a result, the time required for the liquefaction of the simulated highly viscous menstrual blood was 40 minutes. The results are shown in Table 2 below.

### <Comparative Example 2>

### (Napkin Test)

The napkin test was conducted in the same manner as in Example 2, except that nattokinase was not sprayed onto the sanitary napkin. As a result, the remaining amount was 0.85 g. The results are shown in Table 2 below.

### [Table 2]

**Table 2**

| | Object | Functional substance | Result of dropping Test | Result of napkin test |
|---|---|---|---|---|
| | | | (min) | (g) |
| Example 2 | Simulated highly viscous menstrual blood | Nattokinase | 10 | 0.03 |
| Example 3 | Simulated highly viscous menstrual blood | Plasmin | 40 | - |
| Comparative Example 2 | Simulated highly viscous menstrual blood | - | - | 0.85 |

As shown in Table 2, in the case of using the absorbent article according to Example 2 having nattokinase as a functional substance, the solid content of the simulated highly viscous menstrual blood was dissolved, and almost no simulated highly viscous menstrual blood remained on the napkin surface. On the other hand, in a case of using the absorbent article of Comparative Example 2 having no nattokinase, the solid content of the simulated highly viscous menstrual blood was not changed, and the simulated highly viscous menstrual blood remained on the napkin surface. It is considered that in Example 2, nattokinase, which is a functional substance, promoted the liquefaction of the simulated highly viscous menstrual blood, and as a result of which excellent absorbency was observed.

Further, as shown in Table 2, in both cases in which nattokinase and plasmin are used as the functional substances (Examples 2 and 3), it was possible to liquefy the simulated highly viscous menstrual blood. Particularly, in a case where nattokinase was used as the functional substance, the simulated highly viscous menstrual blood could be liquefied at a rate four times higher than that of a case where plasmin was used as the functional substance. This indicates that nattokinase has a higher fibrinolytic ability than plasmin.

### <<Example 4 and Comparative Example 3>>

In Example 4 and Comparative Example 3, experiments were conducted using actual highly viscous menstrual blood collected from humans.

### <Example 4>

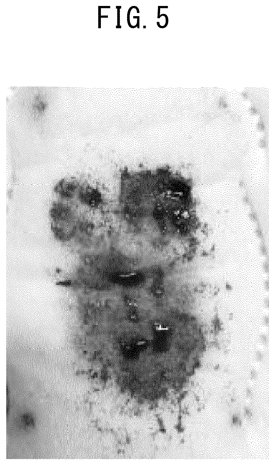

In Example 4, 10 gsm of nattokinase was mixed with PPG-10 methyl glucose of an amphiphilic molecules and was applied onto the top sheet of an absorbent article (trade name: Center-in Happy Catch, manufactured by Unicharm Corporation) and dried. Then, the highly viscous menstrual blood was arranged in the region in which nattokinase was applied, and the absorbent article was left to stand at room temperature for 2 hours. As a result, the liquefaction of the highly viscous menstrual blood was observed. FIG. 4 is a photograph of the napkin surface immediately after the highly viscous menstrual blood was arranged. FIG. 5 is a photograph of the napkin surface after the napkin was allowed to stand at room temperature for 2 hours.

In addition, a test was conducted in which the absorbent articles (n = 2) of Example 4 to which nattokinase had been applied were actually put on. As a result of the test, there was no problem such as itching, and good absorbency was confirmed.

### <Comparative Example 3>

When the highly viscous menstrual blood was arranged on the top sheet of the absorbent article (trade name: Center-in Happy Catch, manufactured by Unicharm Corporation) to which nattokinase was not applied, no liquefaction of the highly viscous menstrual blood was observed even after standing at room temperature for 6 hours.

### <<Examples 5 to 7>>

In Examples 5 to 7, the relationship between the amount of nattokinase applied to the absorbent article and the absorption performance of the absorbent article was examined.

Specifically, in Example 5, 3 mL of horse blood was absorbed in an absorbent article (trade name: Body Fit Regular) coated with nattokinase at a basis weight of 1 gsm and the absorption rate was measured, and further, 3 mL of horse blood was further absorbed and the absorption rate was measured. In addition, the rewetting rate is measured.

It should be noted that in calculating the rewetting rate, after the measurement of the absorption rate, a filter paper (Qualitative Filter Paper N0. 2, manufactured by Advantec Toyo Kaisha, Ltd.) was placed in the region in which horse blood had been added dropwise, and then a load of 30 g/m² was applied and maintained. After 1 minute, the amount of horse blood absorbed into the filter paper was measured and the weight% with respect to the amount of horse blood added dropwise was obtained as the rewetting rate.

As a result, Example 5 showed an absorption rate of 4s or less and a rewetting rate of 20% or less. This absorption performance was equivalent to that of the absorbent article to which nattokinase was not applied.

In Examples 6 and 7, experiments were conducted in the same manner as in Example 5, except that nattokinase was applied at a basis weight of 7 gsm and 12 gsm, respectively. In addition, the rewetting rate is measured.

Examples 6 and 7 also showed an absorption rate of 4s or less and a rewetting rate of 20% or less.

It should be noted that, even in a case where experiments were conducted in the same manner as in Examples 5 to 7, using "HADAOMOI" (trade name, manufactured by Unicharm Corporation) instead of "Body Fit Regular" (trade name, manufactured by Unicharm Corporation) as an absorbent article, similar results were obtained.

### <<Examples 8 to 9>>

Simulated highly viscous menstrual blood (Example 8) or horse blood (Example 9) in the same volume were absorbed, and then experiments were conducted in the same manner as in Example 7 (basis weight: 12 gsm). As a result, in Examples 8 and 9, an absorption rate of 4s or less and a rewetting rate of about 20% were observed.

### <<Examples 10 to 11>>

In Examples 10 to 11, tests were conducted using nattokinase which were heat-treated at a specific temperature condition.

With regard to an absorbent article to which nattokinase heat-treated at 40°C for one week was applied (Example 10), and an absorbent article to which nattokinase without heat treatment was applied (Example 11), evaluations were conducted in the same manner as the napkin test described above with respect to Example 2. As a result, the amount of the simulated highly viscous menstrual blood remaining on each absorbent article was less than 0.10 g. This result shows that even in a case where nattokinase stored at a relatively high temperature is used, good performance of the absorbent article can be obtained.

### <<Examples 12 to 14>>

In Examples 12 to 14, after the application (after the coating) of the nattokinase solution to the absorbent article, a drying treatment or various heat treatments were performed, in order to evaluate the performance of the absorbent article.

With regard to a case where the absorbent article was brought into contact with a metal plate having the temperature of 150°C for 5 seconds immediately after coating (Example 12), a case where the absorbent article was brought into proximity with a metal plate having the temperature of 150°C with a clearance of 1 mm for 1 minute immediately after coating (Example 13), and a case where the absorbent article was dried after coating at 25°C (Example 14), evaluations were conducted in the same manner as the napkin test described above with respect to Example 2. As a result, the amount of the simulated highly viscous menstrual blood remaining on each absorbent article was less than 0.25 g. This result shows that for example, even in a case where nattokinase is applied by a method involving a high temperature, such as hinge roll or HMA coating, good performance of the absorbent article can be obtained.

### REFERENCE SIGNS LIST

1: sanitary napkin
2: top sheet
3: absorbent core
4: side flap
5 side seat
6, 6': embossment
7: application region of functional substance

## Claims

1. An absorbent article (1) for absorbing menstrual blood, having a functional substance, wherein
the functional substance has a function of inhibiting a factor that acts on blood coagulation,
the functional substance is an enzyme having fibrin degradation ability, and
the enzyme is a serine protease.

2. The absorbent article according to Claim 1, wherein
the absorbent article further comprises an auxiliary component capable of enhancing the fibrin degradation ability of the enzyme.

3. The absorbent article according to Claim 1 or 2, wherein
the enzyme having the fibrin degradation ability is nattokinase.

4. The absorbent article according to any one of Claims 1 to 3, wherein
the functional substance contains a compound having an antiplatelet action.

5. The absorbent article according to any one of Claims 1 to 4, wherein
the functional substance is solid or pasty.

6. The absorbent article according to any one of Claims 1 to 5, wherein
the absorbent article is configured so that when the absorbent article is put on, the functional substance contained in the absorbent article is arranged in an excretion opening contact region.

7. The absorbent article according to any one of Claims 1 to 6, further comprising a liquid-permeable top sheet (2) and an absorbent core (3), wherein
at least any one of the top sheet (2) and the absorbent core (3) has the functional substance.

8. The absorbent article according to Claim 7, wherein
the top sheet has the functional substance.

9. The absorbent article according to Claim 7 or 8, wherein
the absorbent core (3) has the functional substance.

10. The absorbent article according to any one of Claims 7 to 9, further comprising a second sheet arranged between the top sheet and the absorbent core, wherein
the second sheet has the functional substance.

## Patentansprüche

1. Absorbierender Artikel (1) zum Absorbieren von Menstruationsblut, der eine funktionelle Substanz aufweist, wobei
die funktionelle Substanz eine Funktion der Inhibierung eines Faktors aufweist, der auf Blutgerinnung einwirkt,
die funktionelle Substanz ein Enzym ist, das Fibrinabbaufähigkeit aufweist, und
das Enzym eine Serinprotease ist.

2. Absorbierender Artikel nach Anspruch 1, wobei
der absorbierende Artikel weiter eine Hilfskomponente umfasst, die die Fibrinabbaufähigkeit des Enzyms verstärken kann.

3. Absorbierender Artikel nach Anspruch 1 oder 2, wobei
das Enzym, das die Fibrinabbaufähigkeit aufweist, Nattokinase ist.

4. Absorbierender Artikel nach einem der Ansprüche 1 bis 3, wobei
die funktionelle Substanz eine Verbindung enthält, die eine thrombozytenhemmende Wirkung aufweist.

5. Absorbierender Artikel nach einem der Ansprüche 1 bis 4, wobei
die funktionelle Substanz fest oder pastenartig ist.

6. Absorbierender Artikel nach einem der Ansprüche 1 bis 5, wobei
der absorbierende Artikel derart konfiguriert ist, dass, wenn der absorbierende Artikel angelegt wird, die funktionelle Substanz, die in dem absorbierenden Artikel enthalten ist, in einem Kontaktbereich der Ausscheidungsöffnung angeordnet ist.

7. Absorbierender Artikel nach einem der Ansprüche 1 bis 6, der weiter eine flüssigkeitsdurchlässige obere Lage (2) und einen Saugkern (3) umfasst, wobei
mindestens eines der oberen Lage (2) und des Saugkerns (3) die funktionelle Substanz aufweist.

8. Absorbierender Artikel nach Anspruch 7, wobei
die obere Lage die funktionelle Substanz aufweist.

9. Absorbierender Artikel nach Anspruch 7 oder 8, wobei
der Saugkern (3) die funktionelle Substanz aufweist.

10. Absorbierender Artikel nach einem der Ansprüche 7 bis 9, der weiter eine zweite Lage umfasst, die zwischen der oberen Lage und dem Saugkern angeordnet ist, wobei
die zweite Lage die funktionelle Substanz aufweist.

## Revendications

1. Article absorbant (1) destiné à absorber le sang menstruel, ayant une substance fonctionnelle, dans lequel
la substance fonctionnelle a pour fonction d'inhiber un facteur qui agit sur la coagulation du sang,
la substance fonctionnelle est une enzyme ayant une capacité de dégradation de la fibrine, et
l'enzyme est une sérine protéase.

2. Article absorbant selon la revendication 1, dans lequel
l'article absorbant comporte par ailleurs un composant auxiliaire en mesure d'améliorer la capacité de dégradation de la fibrine de l'enzyme.

3. Article absorbant selon la revendication 1 ou la revendication 2, dans lequel
l'enzyme ayant la capacité de dégradation de la fibrine est la nattokinase.

4. Article absorbant selon l'une quelconque des revendications 1 à 3, dans lequel
la substance fonctionnelle contient un composé ayant une action antiplaquettaire.

5. Article absorbant selon l'une quelconque des revendications 1 à 4, dans lequel
la substance fonctionnelle est solide ou pâteuse.

6. Article absorbant selon l'une quelconque des revendications 1 à 5, dans lequel
l'article absorbant est configuré de telle sorte que, lorsque l'article absorbant est porté, la substance fonctionnelle contenue dans l'article absorbant est disposée dans une région de contact avec l'ouverture d'excrétion.

7. Article absorbant selon l'une quelconque des revendications 1 à 6, comportant par ailleurs une feuille supérieure perméable aux liquides (2) et une partie centrale absorbante (3), dans lequel
au moins l'une quelconque parmi la feuille supérieure (2) et la partie centrale absorbante (3) a la substance fonctionnelle.

8. Article absorbant selon la revendication 7, dans lequel
la feuille supérieure a la substance fonctionnelle.

9. Article absorbant selon la revendication 7 ou la revendication 8, dans lequel
la partie centrale absorbante (3) a la substance fonctionnelle.

10. Article absorbant selon l'une quelconque des revendications 7 à 9, comportant par ailleurs une deuxième feuille disposée entre la feuille supérieure et la partie centrale absorbante, dans lequel
la deuxième feuille a la substance fonctionnelle.
